# EUROPÄISCHE PATENTANMELDUNG

(11) **EP 0 567 840 A1**
(43) Veröffentlichungstag der Anmeldung: **03.11.1993**
(21) Anmeldenummer: 93106007.3
(22) Anmeldetag: 13.04.1993
(51) Int. Cl.: C08K 5/3462, C09K 21/10, C07D 239/62

(54) **Guanidinbarbiturat und Guanidinthiobarbiturat sowie deren Verwendung in Kunststoffen**

(30) Priorität: 27.04.1992 AT 863/92
(71) Anmelder: DSM Chemie Linz GmbH, 4021 Linz (AT)
(72) Erfinder: Horacek, Heinrich, Dr., A-4048 Puchenau (AT)
(74) Vertreter: Kunz, Ekkehard, Dr.

(57) **Zusammenfassung**

Verwendung von Guanidinbarbituraten und/oder Guanidinthiobarbituraten als Flammhemmer für Kunststoffe, flammfeste Kunststoffe, die Guanidinbarbiturate und/oder Guanidinthiobarbiturate als Flammhemmer enthalten, sowie Guanidinbarbiturat und Guanidinthiobarbiturat.

## Beschreibung

Verwendung von Kunststoffen durch Zusatz von Guanidinbarbituraten und Guanidinthiobarbituraten als Flammhemmer, flammfeste Kunststoffe mit einem Gehalt an Guanidinbarbituraten oder Guanidinthiobarbituraten sowie Guanidinbarbiturat und Guanidinthiobarbiturat.

Die Erfindung betrifft die Verwendung von Guanidinbarbituraten und Guanidinthiobarbituraten als Flammhemmer für Kunststoffe, Kunststoffe, die zur Verbesserung ihrer Flammfestigkeit Guanidinbarbiturate oder Guanidinthiobarbiturate enthalten, sowie Guanidinbarbiturat und Guanidinthiobarbiturat.

Die üblicherweise verwendeten halogenhältigen Flammhemmer zeigen zwar eine gute Wirkung, sie weisen aber den entscheidenden Nachteil auf, daß sie im Brandfall, vor allem im Falle eines längeren Brandes, toxische und korrosive Chlor- und Bromverbindungen freisetzen. Zur Ausschaltung dieser Nachteile wurden bereits halogenfreie Flammschutzmittel eingesetzt, beispielsweise Melamin oder Melamincyanurat (DE-OS 27 40 092).
Melamin weist unter anderem den Nachteil auf, daß es bei der Verarbeitung der Kunststoffe zum Ausblühen neigt, wodurch es teilweise an die Oberfläche wandert und beispielsweise in den Spritzgußformen einen störenden Belag bildet. Melamincyanurat neigt bei der Einarbeitung in den Kunststoff zur Sublimation, wobei der Kunststoff etwas aufschäumt und die Schüttdichte herabgesetzt wird. Die Aufgabe der Erfindung lag darin, neue Substanzen bereitzustellen, die als Flammhemmer für Kunststoffe geeignet sind. Überraschenderweise wurde gefunden, daß Guanidinbarbiturate und Guanidinthiobarbiturate eine gute flammhemmende Wirkung für Kunststoffe zeigen.

Gegenstand der Erfindung ist demnach die Verwendung von Guanidinbarbituraten und Guanidinthiobarbituraten als Flammhemmer für Kunststoffe Ein weiterer Gegenstand der Erfindung sind flammfeste Kunststoffe, die Guanidinbarbiturate und/oder Guanidinthiobarbiturate enthalten.
Guanidinbarbiturate und Guanidinthiobarbiturate können durch Umsetzung von Guanidin oder dessen Salzen mit Barbitursäure, Thiobarbitursäure oder deren Derivaten erhalten werden. Unter Derivaten von Barbitursäure und Thiobarbitursäure sind insbesondere Verbindungen der allgemeinen Formel 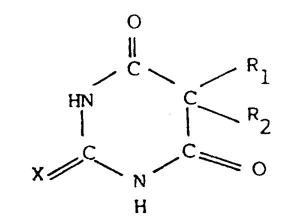
zu verstehen, in denen R₁ und R₂ unabhängig voneinander H, Alkyl- oder Arylreste bedeuten und X für O oder S steht. Diese Verbindungen können beispielsweise durch Reaktion der entsprechend substituierten Malonsäureester mit Harnstoff oder Thioharnstoff erhalten werden. Sie sind auch kommerziell erhältlich. Derivate der Barbitursäure sind beispielsweise in Advances in Heterocylic Chemistry, Academic Press, New York 1985, Vol. 38, Seiten 229 - 241 beschrieben.

Die Umsetzung von Barbitursäure, Thiobarbitursäure oder deren Derivaten mit Guanidin oder dessen Salzen erfolgt bevorzugt in wäßrigem Medium, wobei die schwerlöslichen, substituierten oder unsubstituierten Guanidinbarbiturate bzw. Guanidinthiobarbiturate ausfallen. Diese werden sodann abgetrennt, gewaschen und getrocknet. Bevorzugt wird 1 Mol Guanidin bzw. 1 Äquivalent von Guanidinsalzen, beispielsweise 1/2 Mol Guanidincarbonat, mit 1 Mol Barbitursäure, Thiobarbitursäure oder deren Derivaten umgesetzt. Substituierte Guanidinbarbiturate und Guanidinthiobarbiturate, die als Pharmazeutika verwendet werden, sind beispielsweise in US 2,932,643 beschrieben.

Ein weiterer Gegenstand der Erfindung sind Guanidinbarbiturat und Guanidinthiobarbiturat. Ihre Herstellung erfolgt beispielsweise durch Umsetzung von unsubstituierter Barbitursäure oder Thiobarbitursäure mit Guanidin oder dessen Salzen.
Guanidinbarbiturat besitzt einen Schmelzpunkt von 274 - 278°C, der Zersetzungspunkt liegt bei 430°C, die Löslichkeit in Wasser bei 0,2 g/l (20°C). Der Schmelzpunkt von Guanidinthiobarbiturat liegt bei 320 - 325°C, die Zersetzungstemperatur bei über 450°C, die Löslichkeit in Wasser bei 0,1 g/l (20°C).

Erfindungsgemäß sind Guanidinbarbiturate und Guanidinthiobarbiturate sowohl für thermoplastische als auch für duromere oder elastomere Kunststoffe als Flammhemmer geeignet. Als Kunststoffe kommen beispielsweise solche aus der Gruppe der Polyolefine, wie z. B. Polyethylen, Polypropylen, bzw. Ethylen-Propylen-Copolymere, Polybutylen oder Polymethylpenten, Polyvinylacetat, Polyamide, Polyacrylnitril oder Polyacrylnitril enthaltende Kunststoffe, wie z. B. ABS (Acrylnitril-Butadien-Styrol Copolymer) oder SAN (Styrol-Acrylnitril-Copolymer), thermoplastische oder vernetzte Polyurethane, thermoplastische, ungesättigte oder vernetzte Polyester, Epoxide, Acrylharze, Harnstoff-, Melamin- oder Phenolformaldehydharze in Frage. Die Kunststoffe können auch geschäumt sein. Weiters ist es möglich, Mischungen verschiedener Kunststoffe oder Copolymere aus verschiedenen Monomeren, beispielsweise Ethylen-Propylen-Copolymere, mit Guanidinbarbituraten oder Guanidinthiobarbituraten flammhemmend auszurüsten. Besonders geeignet als Flammhemmer erweisen sich Guanidinbarbiturate und Guanidinthiobarbiturate für stickstoffhältige Kunststoffe, wie z. B. Polyamide, Polyurethane, Polyacrylnitril oder Polyacrylnitril enthaltende Kunststoffe.
Außer ihrer guten flammhemmenden Wirkung besitzen Guanidinbarbiturate und Guanidinthiobarbiturate den zusätzlichen Vorteil, daß sie schwer wasserlöslich sind.

Erfindungsgemäß können Guanidinbarbiturate und Guanidinthiobarbiturate für sich allein oder gemeinsam mit anderen Flammhemmern eingesetzt werden. Als weitere Flammhemmer eignen sich vorzugsweise halogenfreie, wie z. B. Melamin, Melamincyanurat, Flammhemmer auf Phosphorbasis, beispielsweise Ammoniumpolyphosphat, Phosphorsäureester und roter Phosphor oder Flammhemmer auf Basis von Borsäureestern.

Die Herstellung der flammfesten Kunststoffe erfolgt beispielsweise durch Mischen von Guanidinbarbituraten oder Guanidinthiobarbituraten bzw. deren Mischungen mit den jeweiligen Kunststoffen. Im Falle von thermoplastischen Kunststoffen kann die Mischung anschließend beispielsweise in einem Extruder aufgeschmolzen werden. Im Falle von Reaktionsharzen ist es auch möglich, Guanidinbarbiturate und Guanidinthiobarbiturate den Reaktionskomponenten zur Herstellung des Kunststoffes bereits im Zuge seiner Herstellung zuzusetzen. Beispielsweise ist es im Falle von Polyurethanen möglich, Guanidinbarbiturate und/oder Guanidinthiobarbiturate sowie gegebenenfalls weitere Flammhemmer bereits den Polyolen oder den Polyisocyanaten vor der Polymerisationsreaktion zuzusetzen.

Zur flammhemmenden Ausrüstung der Kunststoffe werden bevorzugt Guanidinbarbiturate bzw. Guanidinthiobarbiturate verwendet, bei denen mindestens 95 Gew.% eine Korngröße von maximal 0,025 mm besitzen. Der Gehalt der Kunststoffe an Guanidinbarbituraten bzw. Guanidinthiobarbituraten beträgt entsprechend den jeweiligen Anforderungen an die Flammbeständigkeit üblicherweise etwa 1 bis 30 Gew.%, bevorzugt etwa 5 bis 20 Gew.%.

In den nachfolgenden Beispielen wurden folgende Kunststoffe flammhemmend ausgerüstet:
- PA 6: Polyamid 6 (Ultramid B4, BASF)
- PA6,6: Polyamid 6,6 (Durethane A31, Bayer)
- TPU: Thermoplastisches Polyurethan Desmopan Shore A80, Bayer)
- ABS: Acrylnitril-Butadien-Styrol-Copolymer (Terluran 99S, BASF)
- SAN: Styrol-Acrylnitril-Copolymer (Luran 53, BASF)
- PP: Polypropylen (Daplen PP CS10, Petrochemie Danubia)
- PET: Polyethylenterephthalat (Arnite D02300, Akzo)
- PBT: Polybutylenterephthalat (Arnite T06200, Akzo)

### Beispiel 1:

### Herstellung von Guanidinbarbiturat:

600 g Wasser und 384 g (3 Mol) Barbitursäure (Merck) wurden auf 90°C erhitzt und unter Rühren 270 g (1,5 Mol) Guanidincarbonat (Chemie Linz) zugegeben. Nach weiterem 2-stündigen Rühren bei Siedetemperatur unter Rückfluß wurde das gebildete Guanidinbarbiturat abgesaugt, zweimal mit kaltem Wasser gewaschen und über Nacht bei 105°C im Trockenschrank getrocknet. Guanidinbarbiturat wurde mit einer Ausbeite von 75 % gewonnen. Der Schmelzpunkt lag bei 274-278°C, die Zersetzungstemperatur bei 430°C. Die Verbindung wurde mittels IR-Spektrum charakterisiert.

| Elementaranalyse: | | | | |
|---|---|---|---|---|
| | C | H | N | O |
| theoretisch | 32,1 % | 4,8 % | 37,4 % | 25,7 % |
| gefunden | 31,5 % | 4,9 % | 36,4 % | 26,0 % |

### Beispiel 2:

### Herstellung von Guanidinthiobarbiturat:

600 g Wasser und 432 g (3 Mol) Thiobarbitursäure (Merck) wurden auf 90°C erhitzt und unter Rühren 270 g (1,5 Mol) Guanidincarbonat (Chemie Linz) zugegeben. Nach weiterem 2-stündigen Rühren bei Siedetemperatur unter Rückfluß wurde das gebildete Guanidinthiobarbiturat abgesaugt, zweimal mit kaltem Wasser gewaschen und über Nacht bei 105°C im Trockenschrank getrocknet. Guanidinthiobarbiturat wurde mit einer Ausbeite von 80 % gewonnen. Der Schmelzpunkt lag bei 320-325°C, die Zersetzungstemperatur bei über 450°C. Die Verbindung wurde mittels IR-Spektrum charakterisiert.

| Elementaranalyse: | | | | | |
|---|---|---|---|---|---|
| | C | H | N | O | S |
| theoretisch | 29,5 % | 4,4 % | 34,5 % | 15,8 % | 15,8 |
| gefunden | 29,2 % | 4,5 % | 33,8 % | 16,0 % | 16,5 % |

### Beispiel 3:

Einem Zweischneckenextruder (LSM 30/34 GL 9R, Fa. Leistritz) wurden getrennt voneinander 5 kg/h PA 6 und 0,32 kg/h Guanidinbarbiturat zudosiert, bei 270°C aufgeschmolzen und homogenisiert, über eine 2 mm Lochdüse extrudiert und mittels Kaltabschlag granuliert. Die Granulate wurden anschließend auf einer Heißpresse bei 280°C zu 3,2 mm dicken Plättchen verpreßt, die gemäß UL 94 auf ihre Flammfestigkeit geprüft wurden. Die Flammfestigkeit lag bei V-O, entsprechend einer maximalen Nachbrenndauer von 10 sec nach 10 sec Beflammung.

### Beispiele 4 - 16:

Analog zu Beispiel 3 wurden Probeplättchen aus mit Guanidinbarbiturat (GB) oder Guanidinthiobarbiturat (GTB)flammfest ausgerüsteten Kunststoffgranulaten hergestellt, wobei jedoch die in Tabelle 1 angeführten Kunststoffe und Mengen (Gew.%) Guanidinbarbiturat bzw. Guanidinthiobarbiturat eingesetzt wurden. In Beispiel 9 wurde zusätzlich zum Guanidinbarbiturat Ammoniumpolyphosphat (Exolit 422, Hoechst) als weiterer Flammhemmer mitverwendet. Die Flammfestigkeit entsprach in allen Fällen der Brandklasse V - O.

### Vergleichsbeispiel V1:

Analog zu Beispiel 2 wurden Probeplättchen aus PA6 ohne Flammhemmer hergestellt. Die Flammfestigkeit entsprach der Brandklasse V - 2 gemäß UL94, entsprechend einer Nachbrennzeit von 30 Sekunden, wobei zusätzlich brennende Teilchen abfielen.

### Vergleichsbeispiel V2:

Analog zu Beispiel 2 wurden Probeplättchen aus PA6 mit einem Zusatz von 8 Gew.% Melamincyanurat (Chemie Linz) als Flammhemmer hergestellt. Die Flammfestigkeit entsprach der Brandklasse V-O gemäß UL 94. Das Schüttgewicht der Granulate lag mit 620 g/l (Tabelle 1) unter jenem der mit Guanidinbarbiturat bzw. Guanidinthiobarbiturat ausgerüsteten Kunststoffe.

**Tabelle 1**

| | Kunststoff | Flammhemmer* | Gew.% | Brandklasse (UL 94) | Schüttgewicht der Granulate (g/l) |
|---|---|---|---|---|---|
| 3 | PA 6 | GB | 6 | V - O | 700 |
| 4 | PA 66 | GB | 6 | V - O | 800 |
| 5 | TPU | GB | 10 | V - O | 750 |
| 6 | ABS | GB | 20 | V - O | 720 |
| 7 | SAN | GB | 20 | V - O | 760 |
| 8 | PP | GB | 25 | V - O | 720 |
| 9 | PP | GB | 12,5 | | |
| | | Phosph | 12,5 | V - O | 680 |
| 10 | PA 6 | GTB | 6 | V - O | 710 |
| 11 | PA 66 | GTB | 6 | V - O | 760 |
| 12 | TPU | GTB | 10 | V - O | 720 |
| 13 | PET | GTB | 20 | V - O | 780 |
| 14 | PBT | GTB+GB | 10+10 | V - O | 750 |
| 15 | ABS | GTB | 25 | V - O | 720 |
| 16 | SAN | GTB | 20 | V - O | 690 |
| V1 | PA 6 | - | - | V - 2 | 620 |
| V2 | PA 6 | MC | 8 | V - 0 | 620 |

| | | | | | |
|---|---|---|---|---|---|
| *GB Guanidinbarbiturat GTB Guanidinthiobarbiturat MC Melamincyanurat Phosph Ammoniumpolyphosphat | | | | | |

## Patentansprüche

1. Verwendung von Guanidinbarbituraten und/oder Guanidinthiobarbituraten als Flammhemmer für Kunststoffe.

2. Flammfester Kunststoff, dadurch gekennzeichnet, daß er Guanidinbarbiturate und/oder Guanidinthiobarbiturate enthält.

3. Flammfester Kunststoff gemäß Anspruch 2, dadurch gekennzeichnet, daß er Stickstoff in der Polymerkette enthält.

4. Flammfester Kunststoff gemäß Anspruch 2 oder 3, dadurch gekennzeichnet, daß die Kunststoffe aus Polyamiden, Polyurethanen, Polyacrylnitril oder Polyacrylnitril enthaltenden Kunststoffen bestehen.

5. Flammfester Kunststoff gemäß einem der Ansprüche 2 bis 4, dadurch gekennzeichnet, daß er 5 bis 20 Gew.% Guanidinbarbiturate und/oder Guanidinthiobarbiturate enthält.

6. Flammfester Kunststoff gemäß einem der Ansprüche 2 bis 5, dadurch gekennzeichnet, daß mindestens 95 Gew.% der Guanidinbarbiturate oder Guanidinthiobarbiturate eine Korngröße von maximal 0,025 mm besitzen.

7. Flammfester Kunststoff gemäß einem der Ansprüche 2 bis 6, dadurch gekennzeichnet, daß er zusätzlich weitere Flammhemmer enthält.

8. Verfahren zur Erhöhung der Flammfestigkeit von Kunststoffen, dadurch gekennzeichnet, daß man den Kunststoffen oder den Reaktionskomponenten zur Herstellung der Kunststoffe Guanidinbarbiturate und/oder Guanidinthiobarbiturate als Flammhemmer zusetzt.

9. Guanidinbarbiturat und Guanidinthiobarbiturat

10. Verfahren zur Herstellung von Guanidinbarbiturat und Guanidinthiobarbiturat, dadurch gekennzeichnet, daß man Guanidin oder dessen Salze mit Barbitursäure oder Thiobarbitursäure umsetzt.
